# EUROPÄISCHE PATENTANMELDUNG

(11) **EP 0 982 004 A1**
(43) Veröffentlichungstag der Anmeldung: **01.03.2000**
(21) Anmeldenummer: 98116172.2
(22) Anmeldetag: 27.08.1998
(51) Int. Cl.: A61B 17/12

(54) **Oesophagus-Varizenligator**

(71) Anmelder: Pauldrach, Georg, 30827 Garbsen (DE)
(72) Erfinder: Pauldrach, Georg, 30827 Garbsen (DE)
(74) Vertreter: Arendt, Helmut, Dipl.-Ing.

(57) **Zusammenfassung**

Für ein Endoskop zum Behandeln von Oesophagus-Varizen ist ein Saugzylinder vorgesehen, der durch einen Zugdraht in einer am distalen Ende der Endoskopspirale angeordnete Endoskopendhülse ziehbar ist. Der Saugzylinder trägt mindestens einen, vorzugsweise mehrere zum Einschnüren einer Varize dienende Gummi- oder Ligaturringe. Der Saugzylinder ist an seinem in die Endoskophülse reichenden Ende mit einem elastischen Ring versehen. An seinem distalen Ende sind die Gummi- oder Ligaturringe angeordnet. Die Endoskopendhülse weist an ihrem Innenmantel mehrere parallele Rillen auf, in welche der elastische Ring des Saugzylinders beim Einziehen in die Endoskophülse greift. Dadurch können die gespannt auf dem Saugzylinder vorrätig gehaltenen elastischen Ringe während der Behandlung in exakter Weise vereinzelt werden.

## Beschreibung

Die Erfindung betrifft ein Gerät zum Behandeln von Oesophagus-Varizen, bestehend aus einem Endoskop mit einem mit dem Endoskop verbindbaren Saugzylinder, der durch den Endoskopzugdraht in eine am distalen Ende der Endoskopspirale angeordnete Endoskopendhülse ziehbar ist und wenigstens einem zum Einschnüren einer Varize dienenden Gummiring trägt.

Eine große Gefahr bei Oesophagus-Varizen, das sind Erweiterungen der Speiseröhrenvenen, ist die zum Teil lebensbedrohliche Varizenblutung. Die moderne Medizin verfügt jedoch durch flexible Endoskope über Möglichkeiten therapeutischer Eingriffe, die zuvor nicht ohne Operationen durchzuführen waren. Hierzu gehört die endoskopische Venenverödung durch eine Nadelinjektion und in jüngerer Zeit die Ligatur, die die Blutzirkulation in der Vene unterbindet. Zu diesem Zweck wird am distalen Ende eines flexiblen Endoskops ein Saugzylinder befestigt, durch den es möglich ist, eine Varize hineinzusaugen und über diese einen auf dem Saugzylinder sitzenden, gespannten Gummiring zu stülpen, wodurch die Varize eingeschnürt wird. Nach der Einschnürung der Varize durch Abstreifen des Gummirings vom Saugzylinder wird dieser mit dem Endoskopgerät von der Behandlungsstelle entfernt und mit einem neuen Gummiring für die Einschnürung einer weiteren Varize wieder vor Ort geführt Die Unterbrechung der Behandlung durch Zurückziehen des Gerätes und Wiedereinführung ist zeitaufwendig und für den Patienten belastend. Es sind deshalb bereits Geräte entwickelt worden, die eine Mehrzahl von Gummiringen zum Einschnüren der Varizen speichern. Die Geräte sind jedoch technisch aufwendiger als sogenannte Einfachgeräte, so daß die Herstellung mit entsprechend höheren Kosten verbunden ist.

Der Erfindung liegt die Aufgabe zugrunde, ein Varizenligaturgerät zu schaffen, das in der Lage ist, bei technisch einfacher Ausbildung eine Mehrzahl von elastischen Ringen einsatzfertig gespannt vorrätig zu halten und deren Vereinzelung während der Behandlung in exakter Weise zu garantieren. Die Erfindung löst diese Aufgabe dadurch, daß der Saugzylinder an seinem in die Endoskopendhülse reichenden Ende mit einem elastischen Ring versehen ist, an seinem distalen Ende mehrere Gummiringe trägt und die Endoskopendhülse an ihrem Innenmantel mehrere parallele Rillen aufweist, in welche der elastische Ring des Saugzylinders beim Einziehen in die Endoskopendhülse greift.
Eine polierte Oberfläche des Saugzylinders, auf dem die Gummiringe aufgespannt sind, ist von großem Vorteil, da hierdurch der Haftwiderstand verringert und jeder einzelne Gummiring leichter abstreifbar ist. Vorzugsweise ist als elastischer Ring ein handelsüblicher O-Ring eingesetzt.

Die parallelen Rillen am Innenmantel der Endoskopendhülse dienen als Rastnuten, so daß die Relativbewegung zwischen dem Saugzylinder und der Endoskopendhülse stufenweise exakt erfolgen kann und entsprechend jeder Raststufe ein einzelner Gummi- oder Ligaturring über das freie Ende des Saugzylinders abgeschoben werden kann.

Besonders vorteilhaft ist die Verwendung eines speziell gestalteten Handgriffs, der das exakte Vereinzeln und Abschieben der Ligaturringe nacheinander von dem Saugzylinder unterstützt und erleichtert. Er besteht aus einem pistolenartigen Handgriff, in welchem ein Betätigungsmechanismus angeordnet ist. Dieser umfaßt ein Rädergetriebe, das von Hand betätigbar ist und mit einer Zahnstange in Verbindung steht, an welcher das distale Ende des Endoskopzugdrahtes befestigt ist. Die Betätigung des Rädergetriebes führt zum stufenweisen Einziehen des Saugzylinders in die Endoskopendhülse über kurze Streckenabschnitte, deren Länge gleich der Dicke eines auf dem Saugzylinder angeordneten Ligaturringes ist. Weitere den Erfindungsgegenstand vorteilhaft gestaltende Merkmale sind in den Patentansprüchen angegeben.

In der Zeichnung ist ein Ausführungsbeispiel der Erfindung schematisch dargestellt und nachstehend erläutert. Es zeigen:
- Figur 1: eine Gesamtansicht eines Gerätes mit einem üblichen Endoskopzuggriff,

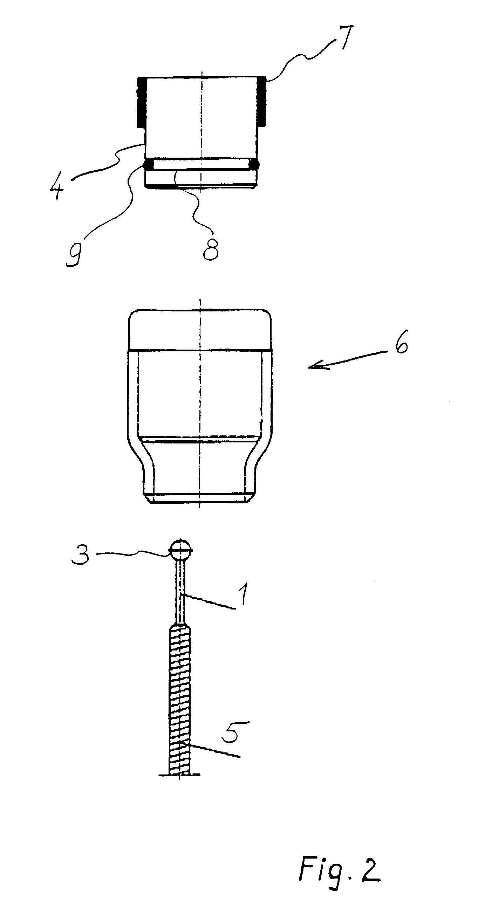

- Figur 2: den Saugzylinder mit Endoskopendhülse und dem distalen Ende des Zugseils, vergrößert dargestellt,
- Figur 3: einen Längsschnitt der Endoskopendhülse mit dem Saugzylinder und fünf Ligaturfingen und dem Abzugseil für eine einsatzfertige Verbindung mit einem Endoskop,

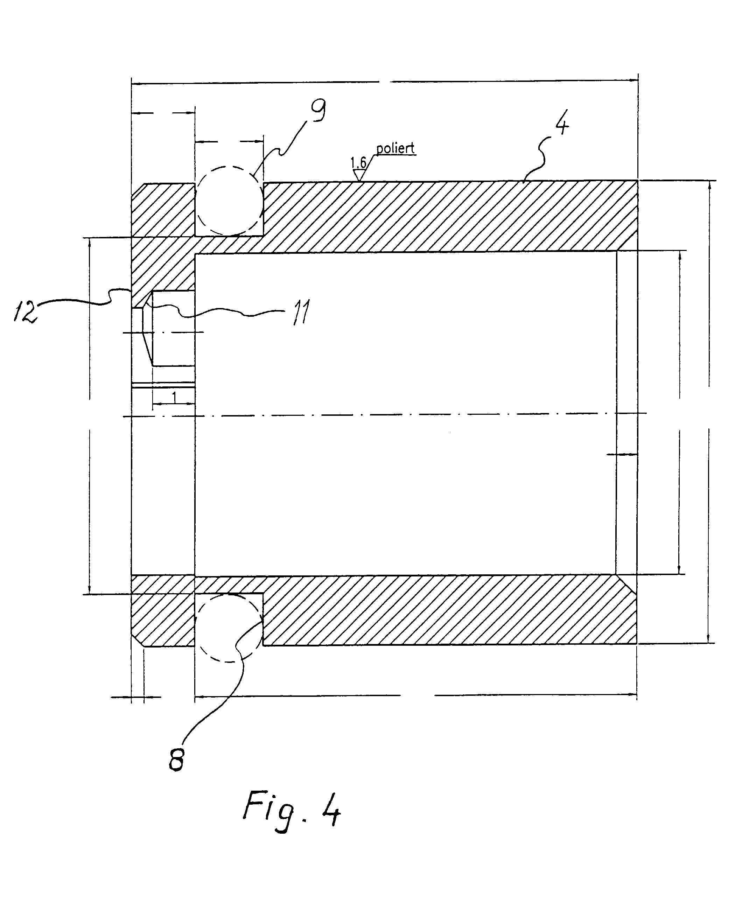

- Figur 4: einen Axialschnitt des Saugzylinders,
- Figur 5: einen Axialschnitt der Endoskopendhülse,

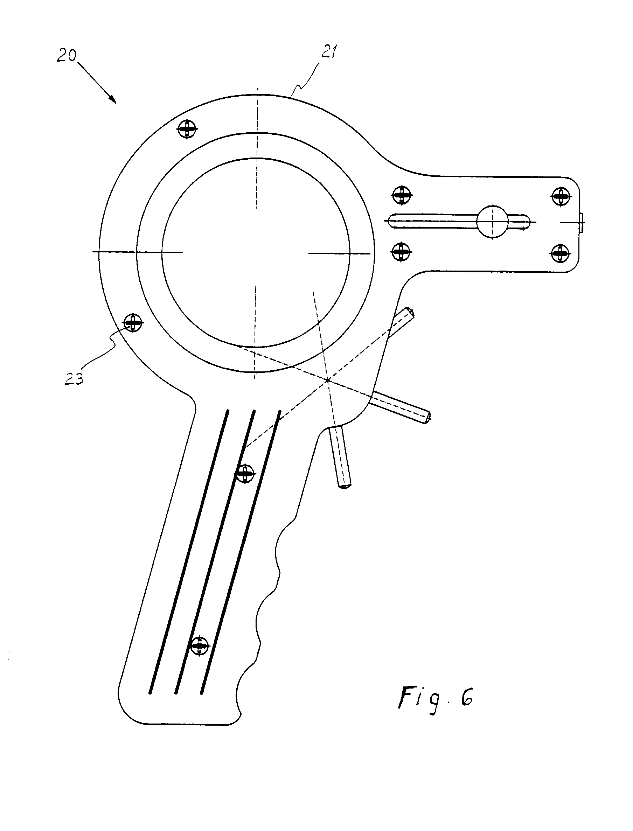

- Figuren 6 und 7: die beiden Außenseiten des pistolenförmigen Gerätehandgnffs,

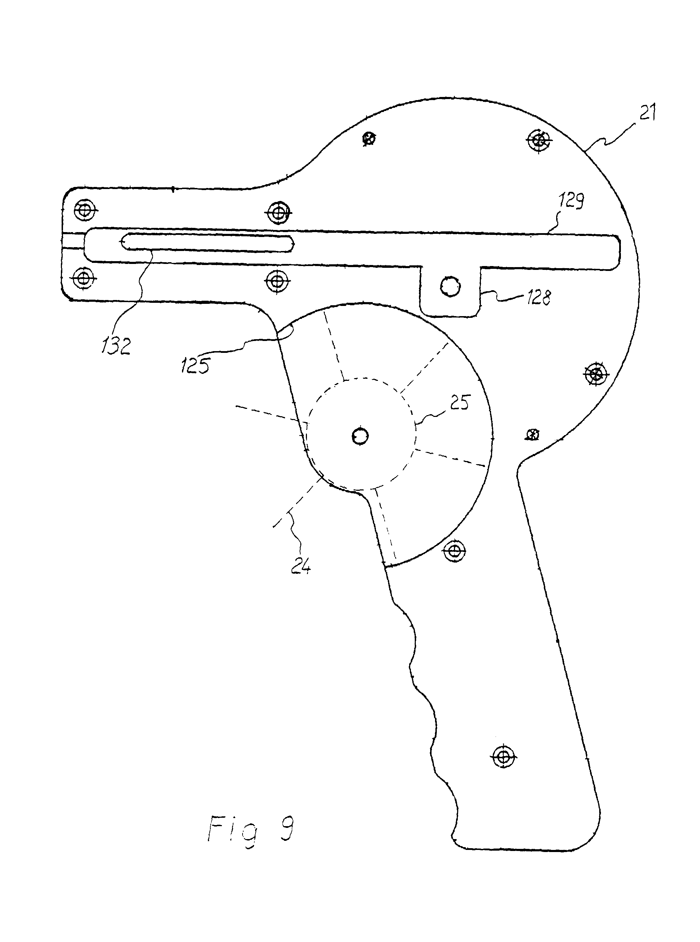

- Figuren 8 und 9: die Innenseiten des Handgriffs nach den Figuren 6 und 7 und

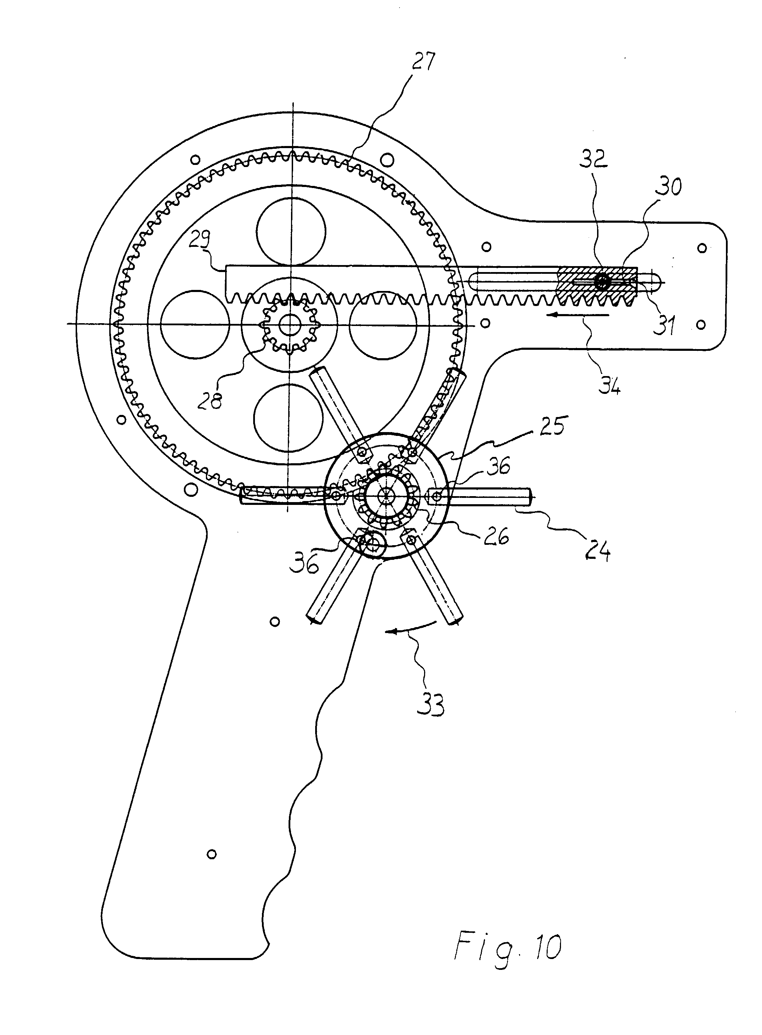

- Figur 10: eine Gehäusehälfte des Handgriffs nach den Figuren 6 bis 9 mit darin angeordneten Getriebeteilen

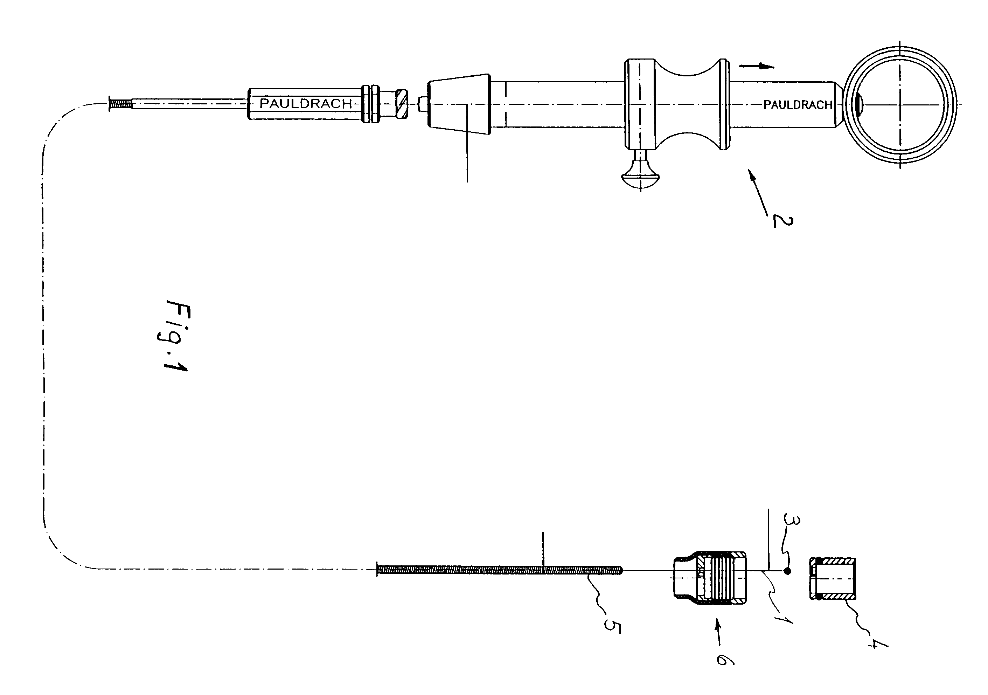

Das Zugseil 1 des Ligators, betätigbar durch einen Handgriff 2 (Fig. 1), trägt an seinem distalen Ende eine Kugel 3, die formschlüssig mit dem Saugzylinder (Ligaturzylinder) 4 verbindbar ist. Das Zugseil ist in der Endoskopspirale 5 geführt und sorgt für das Einziehen des Saugzylinders 4 in die Endoskopendhülse 6.

Die Endoskopendhülse besteht aus einem zylindrischen, distalen Teil 6a aus einem zum Autoklavieren geeigneten Material und einem Adapter 6b aus Silikon, ebenfalls autoklavierbar sowie dehnbar zum Aufschieben auf verschiedene Endoskopdurchmesser.

Der Saugzylinder 4 trägt bei dem dargestellten Beispiel auf seinem distalen Ende maximal fünf gespannte Gummi- oder Ligaturringe 7. In diesem Bereich ist die Oberfläche des Saugzylinders vorzugsweise poliert, um die Haftreibung herabzusetzen.

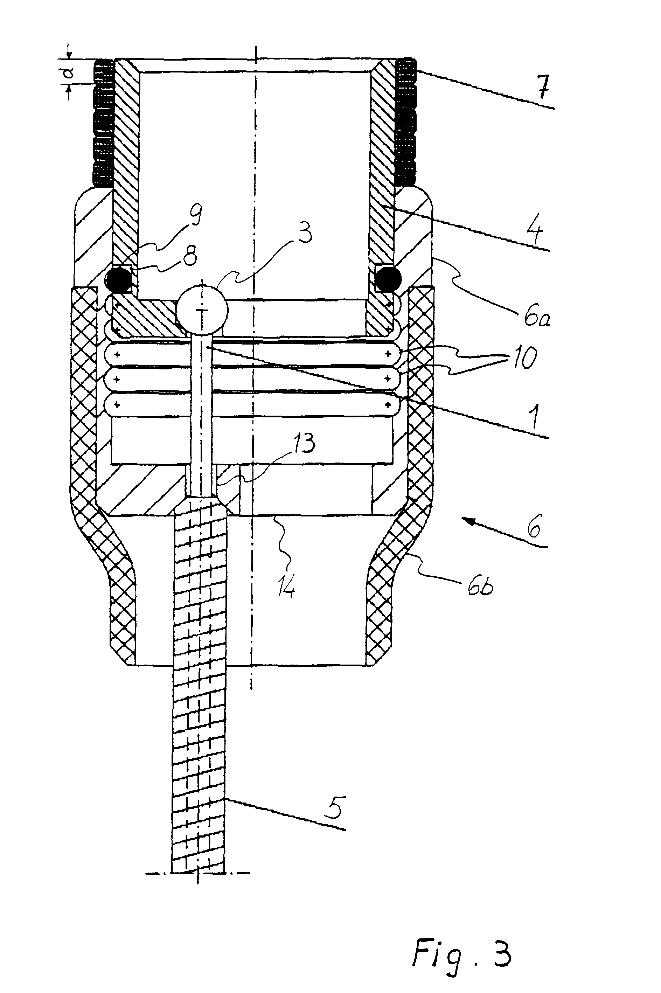

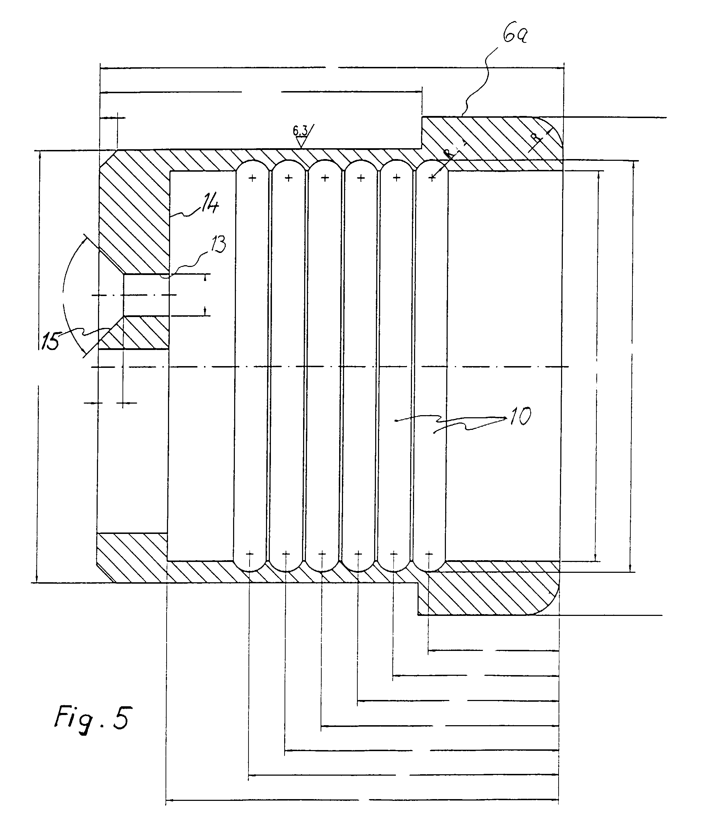

An seinem in die Endoskopendhülse 6 reichenden Ende ist der Saugzylinder 4 mit einer Ringnut 8 versehen, in welche ein O-Ring 9 gelegt ist. Er dient als Rastelement beim Einziehen des Saugzylinders in die Endoskopendhülse. Diese ist zu diesem Zweck mit am Innenumfang angeordneten Rillen 10 (Fig. 3, 5) ausgestattet. Beim Einziehen des Saugzylinders rastet der O-Ring 9 von Rille zu Rille, bei dem gezeigten Beispiel fünf Rillen entsprechend der Anzahl der einzusetzenden Ligaturringe. Durch die Rasteinrichtung wird ein präzises Abstreifen der Ligaturringe 7 vom Außenmantel des Saugzylinders gewährleistet. Es wird vermieden, daß durch zu weites Einziehen des Zylinders mehr als ein Ring abgestreift wird.

Die am distalen Ende des Zugseils 1 angeordnete Kugel 3 ruht in einer Bohrung 11 im Zylinderboden 12 des Saugzylinders. Das Zugseil durchsetzt im montierten Zustand die Bohrung 13 des Zylinderbodens 14 der Endoskopendhülse 6. An ihrem äußeren Ende ist die Bohrung 13 zur Bildung einer Anlagefläche 15 für das distale Ende der Endoskopspirale 5 konisch erweitert.

Mit einem für die Handhabung des Gerätes speziell entwickelten Handgriff 20 wird das Vereinzeln und Einsetzen eines Ligaturrings noch verbessert. Der Handgriff besteht aus einem pistolenförming gestalteten geteilten Gehäuse mit den beiden Gehäusehälften 21 und 22. Die beiden Hälften sind durch Schrauben 23 miteinander verbunden. Die Gehäusehälften sind mit Aussparungen zur Aufnahme der Teile eines Rädergetriebes vorgesehen. Das Rädergetriebe besteht aus einem mit Griffspeichen 24 bestückten Rad 25, das ein koaxial angeordnetes Zahnritzel 26 trägt. Dieses steht mit einem Großrad 27 in Eingriff, das seinerseits mit einem koaxial angeordneten Zahnstangenritzel 28 fest verbunden ist. Das Zahnstangenritzel greift mit seinen Zähnen in die Zähne einer Zahnstange 29. An seinem dem Zahnstangenritzel 28 abgewandten Ende ist die Zahnstange eine Aufnahmebohrung 30, die sich am äußeren Ende 31 konisch erweitert, zur Befestigung des Ligatorzugseils 1 versehen. Dieses ist zu diesem Zweck mit einem nicht dargestellten zylindrischen Ansatz mit einem rotationssymmetrischen Einstich versehen, um das Zugseil formschlüssig mit der Zahnstange zu verbinden. Ein federgelagerter Querriegel 32, der sich in den Einstich am Zugseilende schiebt, sichert den Formschluß mit der Zahnstange.

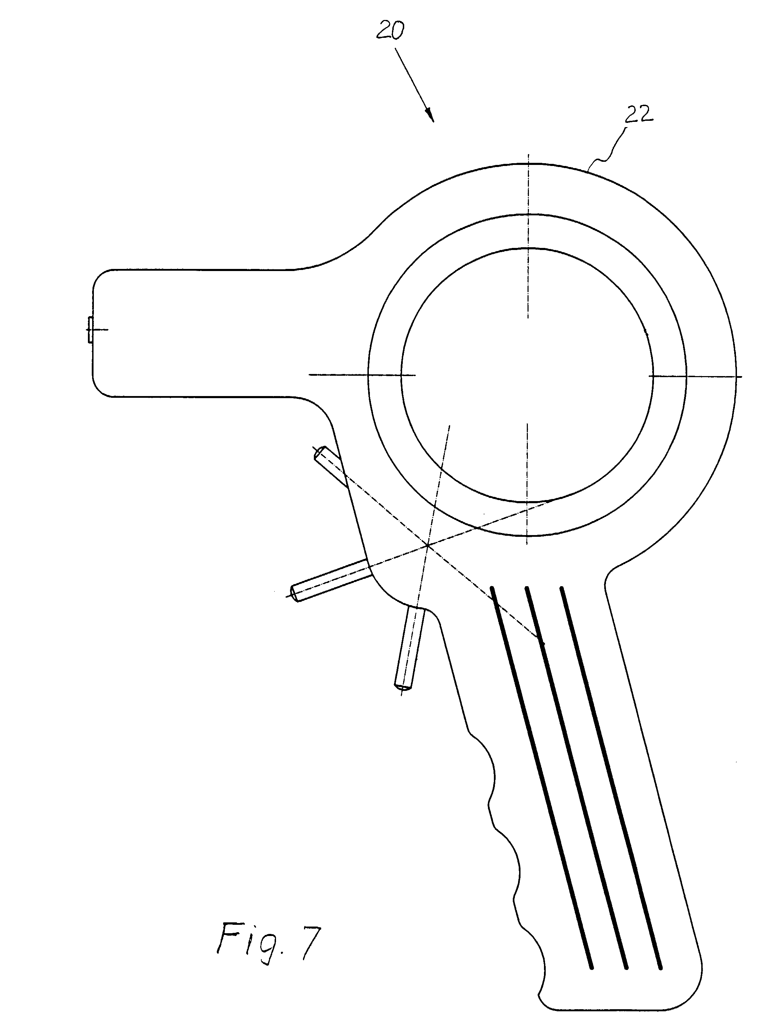

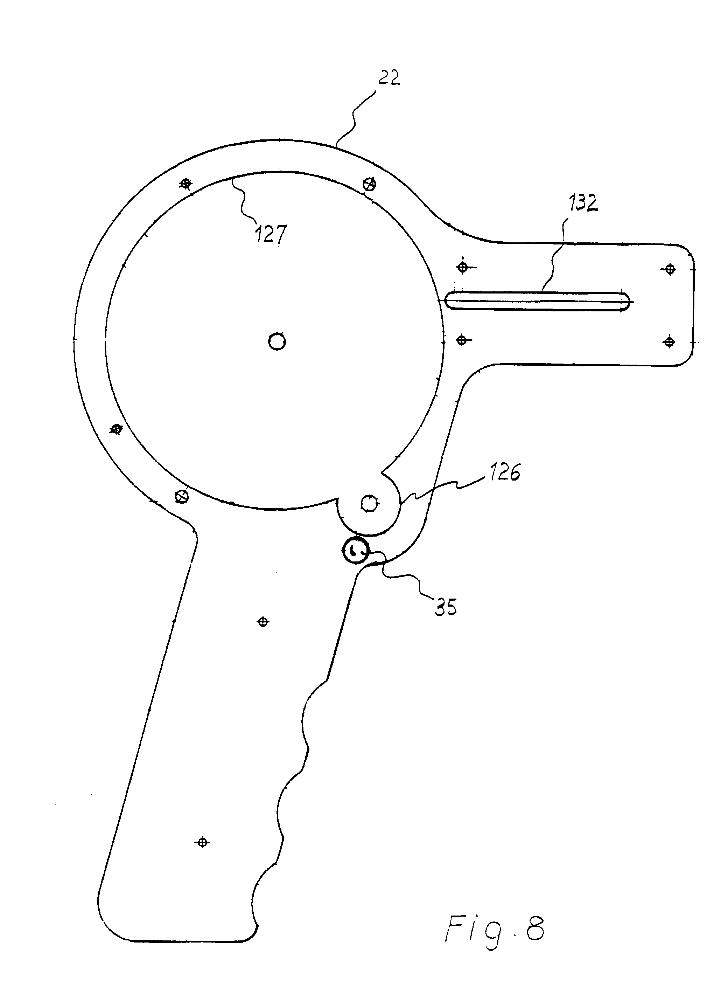

Der von zwei Speichen 24 eingeschlossene Winkel ist in Verbindung mit dem Übersetzungsverhältnis zwischen dem Antriebsritzel 26 und dem Zahnstangenritzel 28 so dimensioniert, daß beim Einschwenken einer Speiche 24 in Richtung des Pfeils 33 bis zum Eintritt in das Gehäuse des Handgriffs 20 die Zahnstange 29 in Richtung des Pfeils 34 um einen Streckenabschnitt verschoben wird, dessen Länge der Dicke eines auf dem Saugzylinder angeordneten Ligaturringes 7 entspricht. Zusätzlich kann in einer Gehäusehälfte eine federnde Rastkugel 35 (Fig. 7 und 8) gelagert sein, die in am Speichenrad 25 angeordnete Mulden 36 einrastet und dadurch der Bedienungsperson zusätzlich das Signal für eine ausreichende Drehung des Speichenrades gibt.

In den Gehäuseteilen 21 und 22 sind die Aussparungen für die Aufnahme des Antriebsritzels 26 mit 126, für das Großrad 27 mit 127, für das Speichenrad 25 mit 125, für das Zahnstangenritzel 28 mit 128 und für die Zahnstange 29 mit 129 bezeichnet. Durch den Öffnungsschlitz 132 greift der federnd gelagerte Befestigunsstift 32 zur formschlüssigen Verbindung des Zugdrahtes 1 mit der Zahnstange 29.

## Patentansprüche

1. Gerät zum Behandeln von Oesophagus-Varizen, bestehend aus einem Endoskop mit einem mit dem Endoskop verbindbaren Saugzylinder, der durch einen Zugdraht in eine am distalen Ende der Endoskopspirale angeordnete Endoskopendhülse ziehbar ist und wenigstens einen zum Einschnüren einer Varize dienenden Gummi- oder Ligaturring trägt, dadurch gekennzeichnet, daß der Saugzylinder (4) an seinem in die Endoskopendhülse (6) reichenden Ende mit einem elastischen Ring (9) versehen ist, an seinem distalen Ende mehrere Gummi- oder Ligaturringe (7) trägt und die Endoskopendhülse an ihrem Innenmantel mehrere parallele Rillen (10) aufweist, in welche der elastische Ring des Saugzylinders beim Einziehen des Saugzylinders in die Endoskopendhülse greift.

2. Gerät nach Anspruch 1, dadurch gekennzeichnet, daß die äußere Oberfläche des Saugzylinders poliert ist.

3. Gerät nach Anspruch 1 oder 2, dadurch gekennzeichnet, daß der elastische Ring ein O-Ring oder ein Metallfederring ist.

4. Gerät nach einem der Ansprüche 1 bis 3, gekennzeichnet durch einen Handgriff (20) mit einem Betätigungsmechanismus zum stufenweisen Einziehen des Saugzylinders (4) in die Endoskopendhülse (6) über kurze Streckenabschnitte gleicher Länge, wobei die Länge eines Streckenabschnitts gleich der Dicke eines auf dem Saugzylinder angeordneten Gummi- oder Ligaturringes (7) ist.

5. Gerät nach Anspruch 4, dadurch gekennzeichnet, daß der Handgriff aus einem geteilten Gehäuse besteht.

6. Gerät nach einem der Ansprüche 1 bis 5, dadurch gekennzeichnet, daß innerhalb des Handgriffgehäuses ein Rädergetriebe (25, 27, 28) angeordnet ist, das mit einer Zahnstange (29) in Wirkverbindung steht, an welcher das distale Ende des Endoskopzugdrahtes (1) gefestigt ist.

7. Gerät nach einem der Ansprüche 1 bis 6, dadurch gekennzeichnet, daß das Antriebsritzel (26) über strahlenförmig angeordnete Griffspeichen (24) betätigbar ist, die zur Bedienung über eine Öffnung des Handgriffgehäuses nach außen weichen.

8. Gerät nach einem der Ansprüche 1 bis 7, dadurch gekennzeichnet, daß durch die Drehung des Speichenrades (25) um einen zwischen den Griffspeichen (24) eingeschlossenen Winkel in Verbindung mit dem Übersetzungsverhältnis der Getrieberäder eine Translationsbewegung der Zahnstange (29) erzielbar ist, die der Dicke (d) eines auf dem Saugzylinder (4) angeordneten Ligaturringes (7) entspricht.

9. Gerät nach einem der Ansprüche 1 bis 8, dadurch gekennzeichnet, daß eine in dem Griffgehäuse angeordnete federnde Rastkugel (35) am Ende einer Drehbewegung des Antriebsritzels in auf dem Speichenrad (25) angeordnete Rastmulden (36) greift.

10. Gerät nach einem der Ansprüche 1 bis 9, dadurch gekennzeichnet, daß die Griffspeichen (24) an einem das Antriebsritzel (26) tragenden Speichenrad (25) befestigt sind und das Antriebsritzel mit einem Großrad (27) in Eingriff steht, welches über ein seitlich koaxial angeordnetes Zahnstangenritzel (28) mit der Zahnstange (29) verbunden ist.
